# EUROPEAN PATENT APPLICATION

(11) **EP 4 362 025 A1**
(43) Date of publication of application: **01.05.2024**
(21) Application number: 22204642.7
(22) Date of filing: 31.10.2022
(51) Int. Cl.: G16B 15/00, G16C 20/30

(54) **METHODS FOR PREDICTION OF TITANIUM DIOXIDE NANOPARTICLES MUTAGENIC AND GENOTOXIC EFFECTS TO HUMAN HEALTH**

(71) Applicant: Qsar Lab Spolka z. o.o, 80-172 Gdansk (PL)
(72) Inventor: Wyrzykowska, Ewelina, 19-300 Elk (PL); Stepnik, Maciej, 94-126 Lodz (PL); Gromelski, Maciej, 82-300 Elblag (PL); Nimz, Kinga, 83-200 Starogard Gdanski (PL); Wojciechowska, Alicja, 06-300 Przasnysz (PL); Puzyn, Tomasz, 80-438 Gdansk (PL)
(74) Representative: JD&P Patent Attorneys Joanna Dargiewicz & Partners

(57) **Abstract**

The invention relates to two methods (method A and B) for prediction of titanium dioxide (TiO₂) nanoparticles mutagenic and genotoxic effects to human health based on Quantitative Structure-Activity Relationships modelling (QSAR). The method A relates to the prediction of mutagenic response that can be used as an equivalent of experimental measurement with *in vitro* micronucleus assay on mammalian cell lines under OECD 487 protocol, whereas method B relates to the prediction of genotoxic response that an equivalent of experimental measurements with *in vitro* comet assay. Both methods were developed with machine learning algorithm called classification decision tree and are based on a set of physicochemical properties (here related to as descriptors) of TiO₂ nanoforms. Method A requires information on three specific descriptors: irregularity of shape, maximum and mean particle size, whereas method B requires information on: irregularity and elongation of shape, maximum, mean and minimum particle size of new TiO₂ nanoforms. The next step is provision of a set of logical rules and their thresholds for each descriptor, within a decision tree classifier for each method. Based on the outcome of the logical tests, a prediction of appropriate response is given as positive or negative. Both methods include domains of applicability (critical thresholds of descriptor values) within which the predictions are high reliability. Provided invention can be used as an alternative to expensive, time-consuming experimental mutagenicity and genotoxicity testing.

## Description

The invention relates to two methods for prediction of titanium dioxide (TiO₂) nanoparticles mutagenic and genotoxic effects to human health based on Quantitative Structure-Activity Relationships modelling (QSAR).

According to the Regulation (EC) No. 1907/2006 of the European Parliament and the Council from December 18, 2006, on the Registration, Evaluation, Authorization and Restriction of Chemicals (REACH), all manufactured or imported chemical substances placed on the European market need to be subjected to a detailed chemical risk analysis in the context of potential exposure scenarios. Due to the estimates of the Joint Research Centre of the European Commission (JRC), which indicated the need to conduct additional experiments with the use of approx. 3.9 million vertebrate animals, an emphasis was put on promoting alternative methods that would allow saving up to 1.9 million animals per year. Taking into account the welfare of laboratory animals, ethical, financial and practical considerations, as well as the reliability of scientific research, a number of alternative methods have been introduced that meet at least one of the principles of the 3R's (replacement, reduction and refinement). Among proposed alternative methods, in silico modelling techniques were indicated, among which Quantitative Structure-Activity Relationship modeling is one of the proposed methods.

Titanium dioxide nanoparticles are manufactured worldwide in large quantities for use in a wide range of applications. TiO₂ nanomaterials possess different physicochemical properties e.g., size distribution, particles' shape, type of coating etc., which might alter their bioactivity e.g., induce inflammation of the respiratory system and gastrointestinal tract. Any alteration of nanoparticle characteristics leads to identification of a new nanoform of this particular substance. For example, TiO₂ nanoparticles distinguished in terms of their size/shape/surface chemistry/etc., will be considered as different TiO₂ nanoforms. The definition of 'nanoform' was introduced in the European Comission Regulation (EU, 2018/1881). Due to different nanoforms inducing different biological effects, there is a need of the safety assessment on animals for each nanoform, which is ethically questionable and financially cost-ineffective. Therefore, computational methods, such as QSAR models serve as an effective alternative to laboratory testing in relation to risk and hazard assessment of nanomaterials to human health, and set basic goal of the invention - to provide methods for prediction of mutagenicity and genotoxicity of TiO₂ nanoforms based on a computer implemented training method, described in the patent application as QSAR models.

The idea behind QSAR modelling is based on quantitative relationships between the structure of chemical substance and its observed activity. The main assumption of this method is that substances with similar structure will exhibit similar biological activity (i.e. cytotoxicity, genotoxicity, mutagenicity). The structure of a substance is characterized numerically by so-called descriptors, that encode substance's molecular and quantummechanical properties calculated with chemoinformatic methods, as well as experimentally measured physicochemical properties. Relationships between descriptors and observed activity can be identified by using supervised machine learning methods. These methods are based on diverse algorithms among which regression and classification analysis can be performed. In case of regression analysis, the predicted response (y) is expressed with continuous values, whereas in classification, the predicted response (y) is categorical (e.g. toxic or non-toxic). The advantage of QSAR modelling is the possibility of identification of underlying processes behind structural features of substances and specific mechanisms of action leading to adverse effects. Moreover, QSAR models can be used to predict unknown activity for new chemical substances solely basing on the descriptors included in the model (X), without the necessity of expensive and time-consuming experimental testing.

The first component of the invention, embodiment is a quantitative structure-activity relationship (QSAR) method for prediction of mutagenic response of TiO₂ nanoparticles (nanoforms), that can be used as an equivalent of experimental measurement with *in vitro* micronucleus assay on mammalian cell lines under OECD 487 protocol.

The method comprises of following steps:
At first, providing a set of selected combination of physicochemical properties of TiO₂ nanoparticles which directly influence the mutagenic response measured with *in vitro* micronucleus assay under OECD 487 protocol that is a set of three specific descriptors required to develop described QSAR model and predict modelled response of new, untested TiO₂ nanoforms:
i. irregular shape that is qualitative factor, derived from Transmission Electron Microscopy (TEM) images defines a measure of nanoparticles irregularity of shape expressed in a binary scale where 1 means irregular and 0 - regular; and
ii. maximum particle size that is quantitative factor, derived from microscopic TEM images defined as maximum size of nanoparticles expressed in nanometers; and
iii. mean particle size that is quantitative factor, derived from microscopic TEM images defined as mean size of nanoparticles expressed in nanometers.

The next step is provision of a set of logical rules and their thresholds for each descriptor, within a decision tree classifier, based on which a prediction of the TiO₂ nanoforms response is performed:
i. [0, 0.5] ≤ irregular shape factor > [0.5, 1]; and
ii. [3.4, 84.0] ≤ maximum particle size [nm] > [84.0, 90.0]; and
iii. [3.4, 34.8] ≤ mean particle size [nm] > [34.8, 90.0]; and
iv. [3.4, 61.0] ≤ maximum particle size [nm] > [61.0, 90.0].

Then providing critical ranges of values for each descriptors that define applicability domain wherein the prediction of modelled mutagenic response is of high reliability:
i. for irregular shape factor - range of values: <0, 1>; and
ii. for maximum particle size - range of values: <3.40, 90> [nm]; and
iii. for mean particle size - range of values: <3.40, 90> [nm].

Finally, prediction of mutagenic response of new, untested TiO₂ nanoforms when values of their descriptors are within the described before critical ranges (i-iii).

The second embodiment is a quantitative structure-activity relationship (QSAR) method for prediction of genotoxic response of TiO₂ nanoparticles that can be used as an equivalent of experimental measurement with *in vitro* comet assay on mammalian cells. The method comprises of following steps.

At first, providing a set of selected combination of physicochemical properties of TiO₂ nanoparticles which directly influence the genotoxic response measured with *in vitro* comet assay protocol that is a set of five specific descriptors required to develop described QSAR model and predict modelled response of new, untested TiO₂ nanoforms:
i. assumed irregular shape factor that is qualitative factor, derived from microscopic TEM images defined as a measure of nanoparticles irregularity of shape expressed in a binary scale (1 - no information about shape, 0 - available information about shape); and
ii. maximum particle size that is quantitative factor, derived from microscopic TEM images defined as maximum size of nanoparticles expressed in nanometers; and
iii. mean particle size that is quantitative factor, derived from microscopic TEM images defined as mean size of nanoparticles expressed in nanometers; and
iv. minimum particle size that is quantitative factor, derived from microscopic TEM images defined as minimum size of nanoparticles expressed in nanometers; and
v. elongation shape factor that is qualitative factor, derived from microscopic TEM images defined as a measure of nanoparticles elongation of shape expressed in a binary scale (1 - elongated shape, 0 - not elongated shape);

Then, providing a set of logical rules and their thresholds for each descriptor, within a decision tree classifier, based on which a prediction of the TiO₂ nanoforms response is performed:
i. [0, 0.5] ≤ assumed irregular shape factor > [0.5, 1]; and
ii. [1.5, 5.69] ≤ mean particle size [nm] > [5.69, 118.0]; and
iii. [1.0, 2.7] ≤ minimum particle size [nm] > [2.7, 65.0]; and
iv. [0, 0.5] ≤ elongated shape factor > [0.5, 1]; and
v. [1.5, 48.0] ≤ maximum particle size [nm] > [48.0, 171.0].

Then, providing critical ranges of values for each descriptors that define applicability domain wherein the prediction of modelled mutagenic response is of high reliability:
i. for assumed irregular shape factor - range of values: <0, 1>; and
ii. for maximum particle size - range of values: <1.50; 171.00> [nm]; and
iii. for mean particle size - range of values: <1.50; 118.00 > [nm]; and
iv. for minimum particle size - range of values: <1.00; 65.00 > [nm]; and
v. for elongation shape factor - range of values: <0, 1>.

Finally, predicting genotoxic response of new, untested TiO₂ nanoforms when values of their descriptors are within the critical ranges (i-v).

According to the invention, provided methods (QSAR models) enable to predict the biological responses - mutagenic and genotoxic effects - of TiO₂ nanoparticles to human health based on selected physicochemical properties known here as descriptors. Provided QSAR models were developed and validated based on experimental measurements for a set of TiO₂ samples (nanoforms) for genotoxic and mutagenic effects. The experimental measurements were performed according to recommended, regulatory relevant guidelines for nanomaterials testing (i.e., micronucleus assay and comet assay, respectively). The experimental data used for models' development were obtained for limited number of TiO₂ samples (nanoforms). However, the identified relationships between the structure (selected descriptors) of preanalyzed TiO₂ nanoforms and observed mutagenic and genotoxic effects determined by the QSAR models' algorithms, allow for estimation of these effects for new TiO₂ nanoforms, instead of experimental testing. The invention, therefore, are methods (QSAR models) for estimation of mutagenic and genotoxic responses of new, untested TiO₂ nanoforms. Predictions from the provided methods are an equivalent of experimental measurements - micronucleus assay (QSAR model for mutagenicity expressed with in vitro micronucleus assay, method A) and comet assay (QSAR model for genotoxicity expressed with in vitro comet assay, method B).

### Detailed explanation

The invention (methods for prediction based on QSAR models) was developed with a use of known machine learning algorithm i.e., classification decision trees, which are constructed basing on a set of logical rules that consider values of specific descriptors in a stepwise manner. During the QSAR models development, the decision tree algorithm identifies a specific set of features (descriptors) relevant for the predicted response and defines appropriate value thresholds for each logical rule that considers particular descriptor. High reliability of predictions is restricted to so-called domains of applicability for each method, which are related to values of selected descriptors used during the calibration of the models. The domains simply describe descriptor value ranges (min/max value thresholds) within which given predictions are reliable.

According to the invention based on QSAR models: metod A and B, prediction of the unknown mutagenic and genotoxic potential of new TiO₂ nanoforms, requires the information about their structural properties i.e., values of descriptors demanded in method A and method B, and then going through a series of logical rules provided in method A and method B, respectively. The assessment of adherence of new TiO₂ nanoparticles to the applicability domain for each model, confirms the reliability of the predictions.

Method A - QSAR model for mutagenicity expressed with in vitro micronucleus assay Toxicity endpoint predicted by the QSAR model: Mutagenic response being an equivalent of experimental measurements with in vitro micronucleus assay according to the regulatory relevant guideline OECD 487 (OECD Guideline for the Testing of Chemicals: In vitro Mammalian Cell Micronucleus Test)
Predicted response: positive/negative mutagenic effect
Application area: nanoforms of titanium dioxide (TiO₂ nanoparticles)

Experimental data used for the QSAR model development - toxicity testing: Nanoforms used for the model development (training set, n=11 nanoforms) and validation (test set, n=5 nanoforms) were experimentally tested in vitro under micronucleus assay (OECD 487). Mutagenicity for each nanoform was experimentally checked on at least one of the following mammalian cell line: PBMC, Caco-2, TK-6, HEK293, NIH/3T3, Balb/c 3T3, L-929, A459, V79.

Experimental data used for the QSAR model development - physicochemical characterization: Nanoforms used for the model development (training set, n=11 nanoforms) and validation (test set, n=5 nanoforms) were experimentally characterized in terms of size distribution and nanoparticles' shape. The size of nanoparticles was measured using a microscope (Transmission Electron Microscopy, abbreviated as TEM), allowing for measurement of their sizes: minimum, average, median and maximum (min, mean, median, max). The shape of nanoparticles was measured using a TEM microscope that allowed to identify whether the nanomaterial had spherical, elongated or irregular shape basing on the obtained microscopic images. In case of missing data related to the shape of analyzed nanoparticle, that particular nanoform was assigned to 'assumed irregular shape' class.

Applied method of modelling: machine learning algorithm - a classification decision tree. The algorithm creates the classification model by building decision tree. Each node in the tree is a specific logical test on an attribute, then each branch descending from that node corresponds to one of the possible values for that attribute. Optimized hyperparameters for the QSAR model are as follows: maximum tree depth = 4, split criterion (function to measure quality of a split) - Gini index, minimum number of samples per split = 2, minimum number of samples to be at a leaf node = 1.

Selected descriptors used in the QSAR model (method A):
a. Irregular shape - qualitative factor, derived from microscopic TEM images - a measure of nanoparticles irregularity of shape expressed in a binary scale where 1 means irregular and 0 - regular;
b. Maximum particle size - quantitative factor, derived from microscopic TEM images - defines maximum size of nanoparticles expressed in nanometers;
c. Mean particle size - quantitative factor, derived from microscopic TEM images - defines mean size of nanoparticles expressed in nanometers.

Applicability domain of the QSAR model: High reliability of predictions for presented QSAR model are restricted to specific critical thresholds of selected descriptors (descriptors' values for nanoforms in the training set that were used to develop the QSAR model). These values simply represent specific ranges for new nanoforms within which any prediction will be reliable. For the method A, the critical thresholds (applicability domain) are presented in Table 1. The cut-off values in Table 1 show where the model domain is effective to correctly predict nanoforms mutagenicity. The minimum and the maximum values of descriptors: 'Maximum particle size' and 'Mean particle size' are 3.40 and 90 nm in both cases. It means that any TiO₂ nanoforms (new, untested samples) within these ranges can be analysed with provided QSAR model and have their mutagenic response predicted correctly with high probability.

**Table 1. Applicability domain of the QSAR model for mutagenicity (method A)**

| **Parameter** | **Minimum value** | **Maximum value** |
|---|---|---|
| Irregular shape factor | 0 | 1 |
| Maximum particle size | 3.40 [nm] | 90 [nm] |
| Mean particle size | 3.40 [nm] | 90 [nm] |

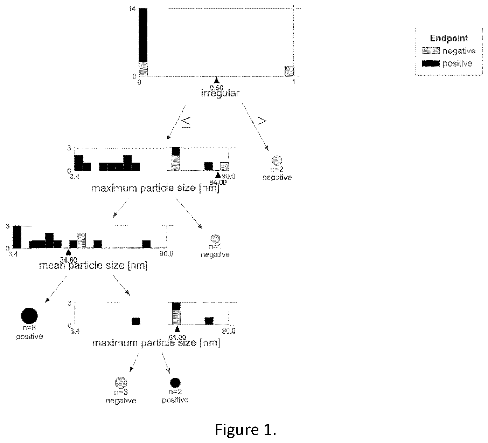

Decision tree classifier for the prediction of mutagenicity: Figure 1 describes a developed QSAR model (method to predict mutagenicity of new, untested TiO₂ nanoparticles). The model is a decision tree with sets of logical rules in each level leading to one of two possible paths (left/right). The invention can be applied as is, without necessity of using advanced software. The scheme leads the user through a logical test in each level to analyze the value of particular descriptor. Based on this value, of currently analyzed descriptor (at current level), the algorithm lets the user follow a further path towards the so-called terminal nodes, that define the final mutagenic response. The example of use of the QSAR model (invention) is described more in detail later in 'Prediction case study A1' and 'Prediction case study A2' sections of the document.

The developed decision tree classifier (QSAR model) resulted in following statistical parameters (Table 2). Presented statistical parameters confirm models' very high goodness-of-fit - a very high convergence between experimentally measured mutagenicity and the predictions of the QSAR model for nanoforms in the training set. Goodness-of-fit is expressed with a set of statistical parameters, i.e. accuracy, precision, recall, f1 score, where the closer each parameter is to 1.0, the better the convergence. For example, an accuracy of 1, describes that 100% of the nanoforms in the training set were correctly classified, namely, the predicted response was the same as the experimental one in all cases. High predictive ability is reflected by a very high convergence between experimentally measured mutagenicity and the predictions of the QSAR model for nanoforms in the test set. Analogously to the goodness-of-fit, predictive ability of the QSAR model was confirmed by high values (equal or close to 1.00) of the same statistical parameters i.e. accuracy, precision, recall and f1 score. For example, an accuracy of 0.80, describes that 80% of the nanoforms in the test set were correctly classified, namely, the predicted response was the same as the experimental one in all cases.

**Table 2. Statistical parameters of presented method for prediction of mutagenic response measured with micronucleus assay**

| **Measure** | **Training set** | **Test set** |
|---|---|---|
| Accuracy | 1.00 | 0.80 |
| Precision | 1.00 | 1.00 |
| Recall | 1.00 | 0.75 |
| F1 score | 1.00 | 0.75 |

Method B - QSAR model for genotoxicity expressed with *in vitro* comet assay Toxicity endpoint predicted by the QSAR model: Genotoxic response being an equivalent of experimental measurements with *in vitro* comet assay
Predicted response: positive/negative genotoxic effect
Application area: nanoforms of titanium dioxide (TiO₂ nanoparticles)

Experimental data used for the QSAR model development - toxicity testing: Nanoforms (samples) used for the model development (training set, n=21 nanoforms) and validation (test set, n=9 nanoforms) were experimentally tested in vitro under comet assay. Genotoxicity for each nanoform was experimentally checked on at least one of the following mammalian cell line: PBMC, Caco-2, HT29, HEK293, NIH/3T3, A459, V79, BEAS-2B, hESCs, 16-HBE, HepG2, HK-2.

Experimental data used for the QSAR model development - physicochemical characterization: Nanoforms used for the model development (training set, n=21 nanoforms) and validation (test set, n=9 nanoforms) were experimentally characterized in terms of size distribution and nanoparticles' shape. The size of nanoparticles was measured using a microscope (Transmission Electron Microscopy, abbreviated as TEM), allowing for measurement of their sizes: minimum, average, median and maximum (min, mean, median, max). The shape of nanoparticles was measured using a TEM microscope that allowed to identify whether the nanomaterial had spherical, elongated or irregular shape basing on the obtained microscopic images. In case of missing data related to the shape of analyzed nanoparticle, that particular nanoform was assigned to 'assumed irregular shape' class.

Applied method of modelling: machine learning algorithm - a classification decision tree. The algorithm creates the classification model by building decision tree. Each node in the tree is a specific logical test on an attribute, then each branch descending from that node corresponds to one of the possible values for that attribute. Optimized hyperparameters for the QSAR model are as follows: maximum tree depth = 3, split criterion (function to measure quality of a split) - Gini index, minimum number of samples per split = 2, minimum number of samples to be at a leaf node = 1.

Selected descriptors used in the QSAR model (method B):
a. Assumed irregular shape factor - qualitative factor, derived from microscopic TEM images - a measure of nanoparticles irregularity of shape expressed in a binary scale (1 - no information about shape, 0 - available information about shape);
b. Maximum particle size - quantitative factor, derived from microscopic TEM images - defines maximum size of nanoparticles expressed in nanometers;
c. Mean particle size - quantitative factor, derived from microscopic TEM images - defines mean size of nanoparticles expressed in nanometers;
d. Minimum particle size - quantitative factor, derived from microscopic TEM images
   - defines minimum size of nanoparticles expressed in nanometers;
e. Elongation shape factor - qualitative factor, derived from microscopic TEM images
   - a measure of nanoparticles elongation of shape expressed in a binary scale (1 - elongated shape, 0 - not elongated shape).

Applicability domain of the QSAR model: High reliability of predictions for presented QSAR model are restricted to specific critical thresholds of selected descriptors (descriptors' values for nanoforms in the training set that were used to develop the QSAR model). These values simply represent specific ranges for new nanoforms within which any prediction will be reliable. For the method B, the critical thresholds (applicability domain) are presented in Table 3. The cut-off values in Table 3 show where the model domain is effective to correctly predict nanoforms genotoxicity. The minimum and the maximum values of quantitative descriptors are as follows: 'Maximum particle size' 1.50 - 171.00 nm, 'Mean particle size' 1.50 - 118.00 nm, 'Minimum particle size' 1.00 - 65.00 nm. It means that any nanoforms (new, untested samples) within these ranges can be analysed with provided prediction method (QSAR model) and have their genotoxic response predicted correctly with high probability.

**Table 3. Applicability domain of the QSAR model for genotoxicity (method B)**

| **Parameter** | **Minimum value** | **Maximum value** |
|---|---|---|
| Assumed irregular shape factor | 0 | 1 |
| Maximum particle size | 1.50 [nm] | 171.00 [nm] |
| Mean particle size | 1.50 [nm] | 118.00 [nm] |
| Minimum particle size | 1.00 [nm] | 65.00 [nm] |
| Elongation factor | 0 | 1 |

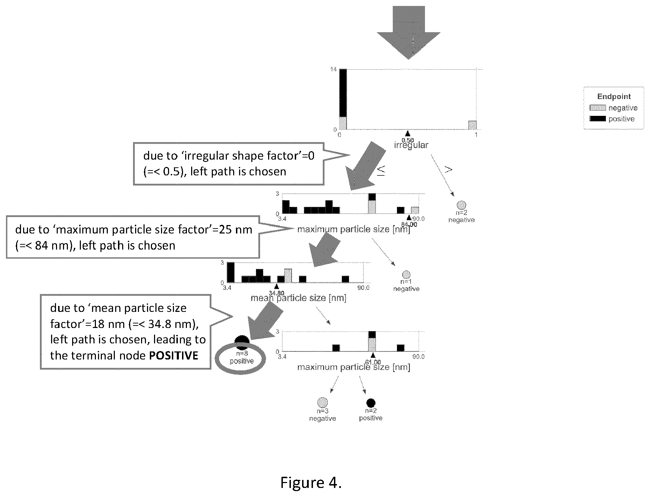

Decision tree classifier for the prediction of genotoxicity: Figure 4 describes a developed QSAR model (method to predict genotoxicity of new, untested TiO₂ nanoparticles). The model is a decision tree with sets of logical rules in each level leading to one of two possible paths (left/right). The invention can be applied as is, without necessity of using advanced software. The scheme leads the user through a logical test in each level to analyze the value of particular descriptor. Based on this value, of currently analyzed descriptor (at current level), the algorithm lets the user follow a further path towards the so-called terminal nodes, that define the final genotoxic response. The example of use of the invention is described more in detail later in 'Prediction case study B1' and 'Prediction case study B2' sections of the document.

The developed decision tree classifier (QSAR model) resulted in following statistical parameters (Table 4). Presented statistical parameters confirm models' very high goodness-of-fit - a very high convergence between experimentally measured genotoxicity and the predictions of the QSAR model for nanoforms in the training set. Goodness-of-fit is expressed with a set of statistical parameters, i.e. accuracy, precision, recall, f1 score, where the closer each parameter is to 1.0, the better the convergence. For example, an accuracy of 0.95, describes that 95% of the nanoforms in the training set were correctly classified, namely, the predicted response was the same as the experimental one in all cases. High predictive ability is reflected by a very high convergence between experimentally measured genotoxicity and the predictions of the QSAR model for nanoforms in the test set. Analogously to the goodness-of-fit, predictive ability of the QSAR model was confirmed by high values (equal or close to 1.00) of the same statistical parameters i.e. accuracy, precision, recall and f1 score. For example, an accuracy of 0.78, describes that 78% of the nanoforms in the test set were correctly classified, namely, the predicted response was the same as the experimental one in all cases.

**Table 4. Statistical parameters of presented method for prediction of genotoxic response measured with comet assay**

| **Measure** | **Training set** | **Test set** |
|---|---|---|
| Accuracy | 0.95 | 0.78 |
| Precision | 0.94 | 1.00 |
| Recall | 1.00 | 0.67 |
| F1 score | 1.00 | 0.67 |

The invention is presented in details in example and drawing:
Figure 1. The scheme of the method A for prediction mutagenicity based on micronucleus assay (OECD 487 guideline);
Figure 2. The scheme of the method B for prediction genotoxicity based on comet assay;
Figure 3. The scheme of the application of method A for prediction mutagenicity for new nanoforms in case study A1;
Figure 4. The scheme of the application of method A for prediction mutagenicity for new nanoforms in case study A2;
Figure 5. The scheme of the application of method B for prediction genotoxicity for new nanoforms in case study B1;
Figure 6. The scheme of the application of method B for prediction genotoxicity for new nanoforms in case study B2.

### Examples

### Introduction

The developed QSAR models are used as methods for prediction of mutagenicity (method A) and genotoxicity (method B) for new, untested TiO₂ nanoforms samples. These methods are derived from the identified relationships between physicochemical properties and adverese effects observed on TiO₂ nanoforms used during the training of the models (nanoforms used in training sets for each method).

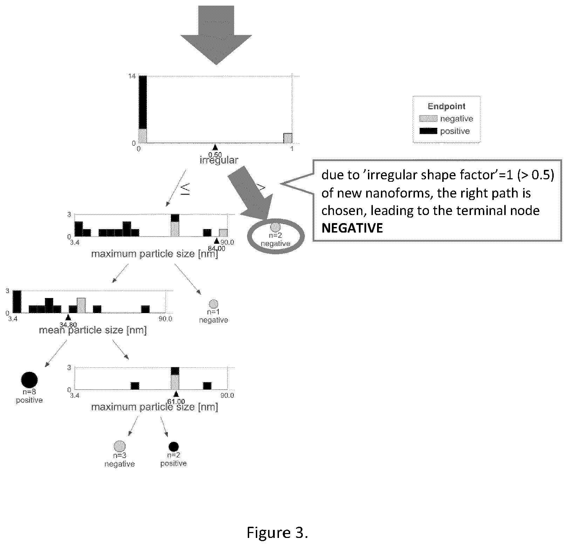

The inventions' use cases are presented in the case studies below. These examples present diverse TiO₂ nanoforms samples for which the application of QSAR models may result in a various responses (positive/negative) for a proper biological effect (mutagenicity, genotoxicity). The application of method A (schematically presented on Fig. 1) was presented on two examples. The first example of mutagenicity prediction with the provided QSAR model (method A) is presented in 'Prediction case study A1' (Example 1, schematically described on Fig. 3), while the second example is presented in 'Prediction case study A2' (Example 2, schematically described on Fig. 4). The case studies A1 and A2 present diverse paths of analysis based on logical rules implemented in method A, and due to different physicochemical properties of new, untested nanoforms (resulting in different values of descriptors demanded in the QSAR model), the outcome of the predictions is 'negative' and 'positive', respectively.

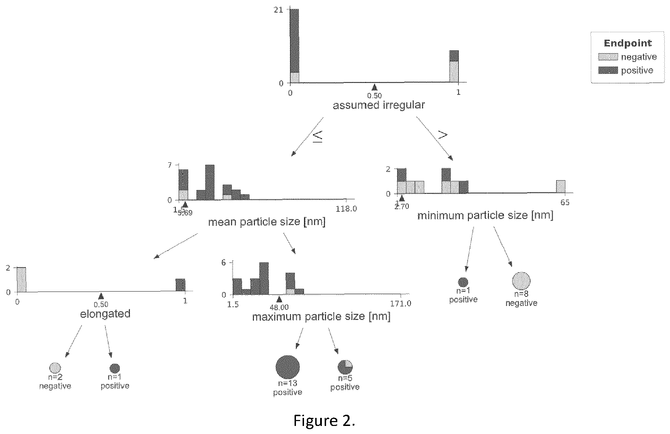

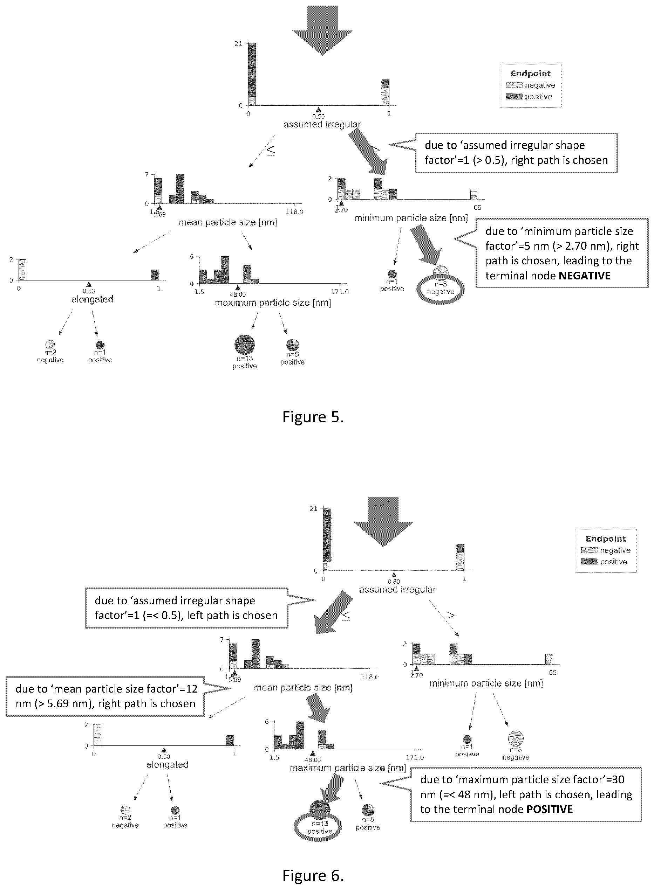

Analogously to the method A, the application of method B (schematically presented on Fig. 2) was also presented on two examples. The first example of genotoxicity prediction with the provided QSAR model (method B) is presented in 'Prediction case study B1' (Example 3, schematically described on Fig. 5), and the second example is presented in 'Prediction case study B2' (Example 4, schematically described on Fig. 6). The case studies B1 and B2 present diverse paths of analysis based on logical rules implemented in method B, and due to different physicochemical properties of new, untested nanoforms (resulting in different values of descriptors demanded in the QSAR model), the outcome of the predictions is 'negative' and 'positive', respectively.

### Prediction case study A1 - Example 1

The presented case study is performed on a new, untested TiO₂ nanoparticle(s) for which the mutagenic effect is unknown. The new TiO₂ nanoparticle(s) are only characterized with known physiochemical parameters, as follow:
irregular shape factor = 1
maximum particle size = 10 nm
mean particle size = 5 nm

The application of method A (QSAR model having a form of the decision tree classifier) requires to follow logical rules of the decision tree from the top to bottom and giving an answer to specific rule. In the first step, the method A requires to assess the 'irregular shape factor' of given nanoparticle(s), i.e. left path when shape factor ≤ 0.5, right path if the shape factor > 0.5. In the presented case study A1, due to nanoparticles' irregular shape ('irregular shape factor' = 1), and according to the first rule, the analyzed nanoparticles are immediately assigned to the right terminal node that defines their mutagenic response as negative (Figure 2).

Predicted mutagenic response: negative

### Prediction case study A2 - Example 2

The presented case study is performed on a new, untested TiO₂ nanoparticle(s) for which the mutagenic effect is unknown. The new TiO₂ nanoparticle(s) are only characterized with known physiochemical parameters, as follow:
irregular shape factor = 0
maximum particle size = 25 nm
mean particle size = 18 nm

The application of method A (QSAR model having a form of the decision tree classifier) requires to follow logical rules of the decision tree from the top to bottom and giving an answer to specific rule. In the first step, the method A requires to assess the 'irregular shape factor' of given nanoparticle(s), i.e. left path when shape factor ≤ 0.5, right path if the shape factor > 0.5. In the presented case study A2, due to nanoparticles' regular shape ('irregular shape factor' = 0), the model does not exclude positive response and takes into account further physicochemical properties (left path) (Figure 3). In the next step, 'maximum particle size' is lower than critical threshold (84 nm), thus again the positive response cannot be excluded (left path again). The next step involves estimation of the nanoparticles' mean size, where any size lower or equal to the critical threshold (34.8 nm) will indicate positive mutagenic response for given nanoparticle(s) (left path and 'positive' terminal node), while for nanoparticles with 'mean particle size' higher than 34.8 nm, the next level of logic rules should be analyzed (right path and value of 'maximum particle size'). As the nanoparticle(s) analyzed in case study A2 have 'mean particle size' = 18 nm, the left path is chosen and 'positive' terminal node is achieved.

Predicted mutagenic response: positive.

### Prediction case study B1 - Example 3

The presented case study is performed on a new, untested TiO₂ nanoparticle(s) for which the genotoxic effect is unknown. The new TiO₂ nanoparticle(s) are only characterized with known physiochemical parameters, as follow:
assumed irregular shape factor = 1
maximum particle size = 10 nm
mean particle size = 5 nm
minimum particle size = 5 nm
elongation factor = 1

The application of method B (decision tree classifier) requires to follow logical rules of the decision tree from the top to bottom and giving an answer to specific rule. In the first step, the method B requires to assess the 'assumed irregular shape factor' of given nanoparticle(s), i.e. left path when shape factor ≤ 0.5, right path if the shape factor > 0.5. In the presented case study B1, due to nanoparticles' unknown shape ('assumed irregular shape factor' = 1), the model takes into account further physicochemical property - minimum particle size (right path) (Figure 5). As the analyzed nanoparticles' 'minimum particle size' = 5 nm (above critical threshold, > 2.70), the right path is chosen and 'negative' terminal node is achieved.

Predicted genotoxic response: negative

### Prediction case study B2 - Example 4

The presented case study is performed on a new, untested TiO₂ nanoparticle(s) for which the genotoxic effect is unknown. The new TiO₂ nanoparticle(s) are only characterized with known physiochemical parameters, as follow:
assumed irregular shape factor = 0
maximum particle size = 30 nm
mean particle size = 12 nm
minimum particle size = 5 nm
elongation factor = 1

The application of method B (decision tree classifier) requires to follow logical rules of the decision tree from the top to bottom and giving an answer to specific rule. In the first step, the method B requires to assess the 'assumed irregular shape factor' of given nanoparticle(s), i.e. left path when shape factor ≤ 0.5, right path if the shape factor > 0.5. In the presented case study B2, due to nanoparticles' known shape ('assumed irregular shape factor' = 0), the model takes into account further physicochemical property - mean particle size (left path). As the analyzed nanoparticles' 'mean particle size' = 12 nm (above critical threshold, > 5.69 nm), the right path is chosen and 'maximum particle size factor' is considered. Due to nanoparticles' 'maximum particle size factor' = 30 nm being lower than the critical threshold (≤ 48 nm), the 'positive' terminal node is achieved.

Predicted genotoxic response: positive.

## Claims

1. A quantitative structure-activity relationship (QSAR) method for prediction mutagenic response of TiO₂ nanoparticles that can be used as an equivalent of experimental measurement with *in vitro* micronucleus assay on mammalian cell lines under OECD 487 protocol, the method comprises of following steps:
b. providing a set of selected combination of physicochemical properties of TiO₂ nanoparticles which directly influence the mutagenic response measured with *in vitro* micronucleus assay under OECD 487 protocol that is a set of three specific descriptors required to develop described QSAR model and predict modelled response of new, untested TiO₂ nanoforms:
iv. irregular shape that is qualitative factor, derived from Transmission Electron Microscopy (TEM) images defines a measure of nanoparticles irregularity of shape expressed in a binary scale where 1 means irregular and 0 - regular; and
v. maximum particle size that is quantitative factor, derived from microscopic TEM images defined as maximum size of nanoparticles expressed in nanometers; and
vi. mean particle size that is quantitative factor, derived from microscopic TEM images defined as mean size of nanoparticles expressed in nanometers;
c. providing a set of logical rules and their thresholds for each descriptor, within a decision tree classifier, based on which a prediction of the TiO₂ nanoforms response is performed:
i. [0, 0.5] ≤ irregular shape factor > [0.5, 1]; and
ii. [3.4, 84.0] ≤ maximum particle size [nm] > [84.0, 90.0]; and
iii. [3.4, 34.8] ≤ mean particle size [nm] > [34.8, 90.0]; and
iv. [3.4, 61.0] ≤ maximum particle size [nm] > [61.0, 90.0];
d. providing critical ranges of values for each descriptor that define applicability domain wherein the prediction of modelled mutagenic response is of high reliability:
i. for irregular shape factor - range of values: <0, 1>; and
ii. for maximum particle size - range of values: <3.40, 90> [nm]; and
iii. for mean particle size - range of values: <3.40, 90> [nm];
d. predicting mutagenic response of new, untested TiO₂ nanoforms when values of their descriptors are within the critical ranges (i-iii).

2. A quantitative structure-activity relationship (QSAR) method for prediction genotoxic response of TiO₂ nanoparticles that can be used as an equivalent of experimental measurement with *in vitro* comet assay on mammalian cells, the method comprises of following steps:
b. providing a set of selected combination of physicochemical properties of TiO₂ nanoparticles which directly influence the genotoxic response measured with *in vitro* comet assay protocol that is a set of five specific descriptors required to develop described QSAR model and predict modelled response of new, untested TiO₂ nanoforms:
vi. assumed irregular shape factor that is qualitative factor, derived from microscopic TEM images defined as a measure of nanoparticles irregularity of shape expressed in a binary scale (1 - no information about shape, 0 - available information about shape); and
vii. maximum particle size that is quantitative factor, derived from microscopic TEM images defined as maximum size of nanoparticles expressed in nanometers; and
viii. mean particle size that is quantitative factor, derived from microscopic TEM images defined as mean size of nanoparticles expressed in nanometers; and
ix. minimum particle size that is quantitative factor, derived from microscopic TEM images defined as minimum size of nanoparticles expressed in nanometers; and
x. elongation shape factor that is qualitative factor, derived from microscopic TEM images defined as a measure of nanoparticles elongation of shape expressed in a binary scale (1 - elongated shape, 0 - not elongated shape);
c. providing a set of logical rules and their thresholds for each descriptor, within a decision tree classifier, based on which a prediction of the TiO₂ nanoforms response is performed:
vi. [0, 0.5] ≤ assumed irregular shape factor > [0.5, 1]; and
vii. [1.5, 5.69] ≤ mean particle size [nm] > [5.69, 118.0]; and
viii. [1.0, 2.7] ≤ minimum particle size [nm] > [2.7, 65.0]; and
ix. [0, 0.5] ≤ elongated shape factor > [0.5, 1]; and
x. [1.5, 48.0] ≤ maximum particle size [nm] > [48.0, 171.0];
d. providing critical ranges of values for each descriptor that define applicability domain wherein the prediction of modelled mutagenic response is of high reliability:
i. for assumed irregular shape factor - range of values: <0, 1>; and
ii. for maximum particle size - range of values: <1.50; 171.00> [nm]; and
iii. for mean particle size - range of values: <1.50; 118.00 > [nm]; and
iv. for minimum particle size - range of values: <1.00; 65.00 > [nm]; and
v. for elongation shape factor - range of values: <0, 1>;
e. predicting genotoxic response of new, untested TiO₂ nanoforms when values of their descriptors are within the critical ranges (i-v).
